# FASCICULE DE BREVET EUROPEEN

(11) **EP 1 021 391 B1**
(45) Date de publication et mention de la délivrance du brevet: **10.12.2003**
(21) Numéro de dépôt: 96937382.8
(22) Date de dépôt: 31.10.1996
(51) Int. Cl.: C07C 37/14, C07C 37/16, C07C 39/08

(54) **PROCEDE DE C-ALKYLATION DE COMPOSES AROMATIQUES HYDROXYLES**
VERFAHREN ZUR C-ALKYLIERUNG VON AROMATISCHEN HYDROXYVERBINDUNGEN
METHOD FOR C-ALKYLATING HYDROXYLATED AROMATIC COMPOUNDS

(30) Priorité: 31.10.1995 FR 9513110
(43) Date de publication de la demande: 26.07.2000
(73) Titulaire: RHODIA CHIMIE, 92408 Courbevoie Cédex (FR)
(72) Inventeur: SERRANO, Cécile, F-69100 Villeurbanne (FR); DURY, Michel, F-69006 Lyon (FR); DESMURS, Jean-Roger, F-60360 St.-Symphorien-d'Ozon (FR); CRUCIANI, Paul, F-69007 Lyon (FR)
(74) Mandataire: Colombet, Alain André
(86) Numéro de dépôt international: FR9601722
(87) Numéro de publication internationale: WO97016402

(56) Documents cités:
- GB-A- 1 469 896
- US-A- 3 373 210
- US-A- 4 461 916
- CHEMICAL ABSTRACTS, vol. 62, no. 6, 15 Mars 1965 Columbus, Ohio, US; abstract no. 6434a, colonne 6434; XP002008358 cité dans la demande & CS 111 292 A (J. POSPISIL, ET AL.)
- COLLECTION OF CZECHOSLOVAK CHEMICAL COMMUNICATIONS, vol. 29, no. 2, Février 1964, PRAGUE, CS, pages 381-389, XP002008357 J. POSPISIL, ET AL.: "Alkylierung von Hydrochinon mit Isobutylen und Diisobutylen" cité dans la demande
- CHEMICAL ABSTRACTS, vol. 119, no. 13, 27 Septembre 1993 Columbus, Ohio, US; abstract no. 138864s, page 838; XP002008359 cité dans la demande & CS 273 290 A
- COLLECTION OF CZECHOSLOVAK CHEMICAL COMMUNICATIONS, vol. 31, no. 1, 1966, PRAGUE, CS, pages 98-105, XP002024507 J. POPISIL, ET AL.: "Antioxydantien und Stabilisatoren X. Bermerkung zur Darstellung einiger Hydrochinon-alkylderivate"
- JOURNAL OF THE AMERICAN CHEMICAL SOCIETY, vol. 64, no. 4, Avril 1942, WASHINGTON, DC, US, pages 937-940, XP002024508 P.F. OESPER, ET AL.: "The reduction of dipole moment by steric hindrance in di-t-butylhydroquinone and its dimethyl ether"
- COLLECTION OF CZECHOSLOVAK CHEMICAL COMMUNICATIONS, vol. 34, no. 7, 1969, PRAGUE, CS, pages 1991-2001, XP002024509 I. BUBEN, ET AL.: "Oxidation of pyrocatechol. VII. Oxidation of 4-tert-alkylpyrocatechols and 2-tert-alkylhydroquinones by oxygen in an alkaline medium"
- COLLECTION OF CZECHOSLOVAK CHEMICAL COMMUNICATIONS, vol. 30, no. 4, 1965, PRAGUE, CS, pages 1092-1103, XP002024510 J. POPISIL, ET AL.: "Antioxydantien und Stabilisatoren V. Darstellung von tert-Butyl- und tert-Octylderivaten des Brenzcatechins"
- CHEMICAL ABSTRACTS, vol. 117, no. 9, 31 Août 1992 Columbus, Ohio, US; abstract no. 89901z, S.D. KIM, ET AL.: "A study on the tert-butylation of hydroquinone" page 743; XP002024511 & HWAHAK KOGHAK, vol. 30, no. 1, 1992, pages 18-25,
- CHEMICAL ABSTRACTS, vol. 67, no. 5, 31 Juillet 1967 Columbus, Ohio, US; abstract no. 21664t, page 2049; XP002024512 & CS 119 828 A (J. POPISIL, ET AL.)

## Description

La présente invention a trait à un nouveau procédé de C-alkylation de composés aromatiques hydroxylés ayant au moins un atome d'hydrogène en ortho ou para par rapport au groupement hydroxylé. Elle concerne en particulier la préparation de 2,5-di-tert-octylhydroquinone.

La synthèse de la 2,5-di-tert-octyl hydroquinone par réaction entre hydroquinone et diisobutylène, comme plus généralement de composés aromatiques hydroxylés ayant au moins un atome d'hydrogène en ortho ou para par rapport au groupement hydroxylé, a fait l'objet de nombreuses variantes au niveau des conditions réactionnelles afin d'en améliorer le rendement.

Le brevet CS n° 111 292 propose l'alkylation de 2-méthylhydroquinone, 2-ter-butylhydroquinone et 2-tert-octylhydroquinone par du diisobutylène avec catalyse par l'acide sulfurique concentré en présence de solvants inertes (chloroforme) ou de solvants réactifs tels que acide acétique glacé ou excès de diisobutylène. Les rendements annoncés sont de l'ordre de 35 %. Voir aussi dans le même sens J. Pospisil et L. Taimr, dans Collect. Czech. Chem. Commun. 29 (1964), 381-5, ou encore J. Pospisil et coll., Collect. Czech. Chem. Commun., 31(1966), 98-105.

Le brevet GB-A-1 469 896 décrit un procédé de synthèse d'un hydroxy-(1,1,3,3-tétraméthylbutyl)-benzène par réaction entre un hydroxybenzène ayant un ou plusieurs groupes hydroxy et étant éventuellement substitué, notamment l'hydroquinone, et un 2,4,4-triméthylpentène, c'est-à-dire l'un de ses isomères α-diisobutylène et β-diisobutylène, ou un mélange des deux, en présence d'acide sulfurique hautement concentré et d'éthylèneglycol. Par acide sulfurique hautement concentré, il est question dans ce brevet d'une concentration dans l'eau supérieure à 90 % et de préférence de l'ordre de 95 ± 3 % . Le rendement annoncé est de l'ordre de 60 % à partir de l'hydroquinone.

Le brevet CS 273 290 propose de préparer la 2,5-bis-(1,1,3,3-tétraméthylbutyl) hydroquinone par alkylation de l'hydroquinone par du diisobutylène avec l'acide sulfurique comme catalyseur, dans un mélange de méthanol et d'hydrocarbures aliphatiques. Le rendement annoncé est de l'ordre de 65 %.

Enfin, le brevet US-A-3 373 210 propose de conduire la réaction d'alkylation en présence de méthanol et avec de l'acide sulfurique comme catalyseur, pour un rendement annoncé de 25 %.

Dans la pratique, il s'avère souvent difficile d'obtenir les rendements annoncés dans ces documents. En outre, des problèmes de polymérisation non négligeables sont rencontrés.

Le besoin existe donc toujours d'un procédé de synthèse conduisant de manière simple et reproductible à des rendements intéressants, qui plus est en minimisant les risques de polymérisation.

L'invention a pour objectif de proposer un tel procédé.

La présente invention a donc pour objet un procédé de C-alkylation d'un composé aromatique hydroxylé ayant au moins un atome d'hydrogène en ortho ou para par rapport au groupement hydroxylé, procédé dans lequel on met ce composé aromatique en présence d'un acide protonique fort ayant un pKa dans l'eau inférieur à -0,1 et d'un composé conduisant à la formation d'un carbocation en présence de l'acide, la réaction étant conduite en présence d'un solvant formé d'un couple eau/alcool, le rapport eau/alcool (en % volume) du solvant étant compris entre 40/60 et 60/40, de préférence 50/50.

Le terme "aromatique" est pris ici dans sa notion classique telle que définie dans l'ouvrage de Jerry March, Advance Organic Chemistry, 4ème édition, Ed. John wiley and Sons, 1992).

Le composé aromatique de départ sera ci-après désigné par hydroxybenzène. Il pourra notamment avoir la formule : 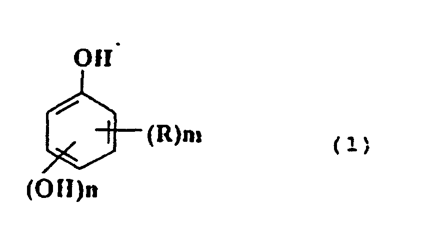 dans laquelle
- n = 0 à 4, notamment 0 ou 1, de préférence 1
- m = 0 à 4, de préférence 0 ou 1
- avec n + m ≤ 4
- R est choisi parmi le groupe consistant en :
   . alkyle linéaire ou ramifié en C1 à C30, notamment en C1 à C6, de préférence en C1 à C4, pouvant éventuellement présenter un ou plusieurs hétéroatomes (Cl, Br, O, N) par exemple CF3,
   . un ou plusieurs alkyles reliés au noyau aromatique par un hétéroatome (N, S, O), l'alkyle répondant à la définition précédente, par exemple un groupe alcoxy, notamment en C1 à C4, de préférence en C1 à C2 par exemple aussi méthylsulfure ou diméthylamine,
   . un atome d'halogène, de préférence Cl, Br.

Il pourra aussi s'agir de composés à noyau hétéroaromatique, par exemple hydroxypyridine, ou de composés aromatiques bicycliques.

Notamment, dans le cas de la formule (1), 2 groupes R placés sur 2 atomes de carbone voisins peuvent former ensemble et avec les atomes de carbone qui les portent 1 cycle aromatique éventuellement substitué.

Par formation de carbocation, il faut comprendre formation de carbocation avec ou sans possibilité de réarrangement. Les composés susceptibles de former une carbocation en présence de l'acide sont de préférence choisis parmi le groupe consistant en :
- composés oléfiniques au moins en C3, notamment en C3 à C30;
- alcools secondaires et tertiaires ; et
- éthers et amines ayant au moins un groupement secondaire ou tertiaire; il peut s'agir en particulier d'éthers ou amines symétriques ou asymétriques ayant au moins un groupement alkyle secondaire ou tertiaire relié à l'oxygène.

Le solvant selon l'invention est formé d'un couple alcool, eau dans lequel l'alcool est préférentiellement un alcool inférieur, notamment choisi dans le groupe consistant en méthanol, éthanol, isopropanol, cyclohexanol et éthylèneglycol, ou mélange d'au moins deux d'entre eux. Le méthanol est l'alcool préféré.

Conformément à l'invention, un solvant formé d'alcool et d'eau doit être présent. Eau et alcool formant ce couple seront en général ajoutés lors de la mise en oeuvre de la réaction. Dans certains cas cependant, lorsque des éthers ou des alcools seront utilisés à titre de source de carbocation, eau et/ou alcool du solvant pourront être générés in situ en tout ou partie à partir des réactifs éther ou alcool.

Le rapport eau/alcool (en % volume) dans le solvant est compris entre 40/60 et 60/40. Un rapport 50/50 est particulièrement approprié, notamment dans le cas où l'alcool est le méthanol. Le solvant eau + alcool sera de préférence utilisé à raison de 1 à 10 ml, plus préférentiellement de 1 à 5 ml, pour 1 g d'acide. Suivant une modalité intéressante de l'invention, on pourra utiliser environ 1 ml d'eau et 1 ml d'alcool, notamment méthanol, pour 1 g d'acide.

Le rapport eau + alcool/acide pourra être généralement compris entre 1/0,1 et 1/10, de préférence entre 1/0,5 et 1/5, exprimé en volume (ml)/masse (g).

L'acide pourra être utilisé à raison de 0,05 à 4 équivalents pour 1 équivalent d'hydroxybenzène.

Le composé oléfinique peut être un hydrocarbure aliphatique insaturé, linéaire ou ramifié, comportant au moins une double liaison de préférence en position α.

Le nombre d'atomes de carbone peut être très variable. Il se situe de préférence entre 3 et 30 atomes de carbone.

Le composé oléfinique peut être également un hydrocarbure monocyclique, carbocyclique, insaturé, ou un hydrocarbure polycyclique comprenant au moins deux carbocycles, pouvant présenter un ou des hétéroatomes dans le cycle et présentant au moins une insaturation endocyclique ou exocyclique.

Il peut y avoir présence dans le cycle de 1 à 2 insaturations. Il est à noter que la double liaison peut être également exocyclique.

Le composé oléfinique peut aussi être un composé terpénique.

Lorsqu'il s'agit d'un hydrocarbure monocyclique, le nombre d'atomes de carbone dans le cycle peut varier largement de 4 à 20 atomes de carbone mais il est de préférence de 5 ou 6 atomes de carbone.

L'hydrocarbure peut être également polycyclique, de préférence bicyclique, ce qui signifie qu'au moins deux cycles ont deux atomes de carbone en commun.

Dans le cas des hydrocarbures polycycliques, la condensation en carbone de chaque cycle est plus faible, généralement de 3 à 8, et est égale de préférence à 5 ou 6 atomes de carbone.

Il est à noter que tout cycle peut porter un ou plusieurs substituants.

Le nombre de substituants présents sur le cycle dépend de la condensation en carbone du cycle et de la présence ou non d'insaturations sur le cycle.

Le nombre maximum de substituants susceptibles d'être portés par un cycle est aisément déterminé par l'Homme du Métier.

Généralement, le nombre de substituants présents sur un cycle est de 1 à 3, de préférence 1 ou 2.

En ce qui concerne la nature des substituants, les exemples de substituants donnés pour R conviennent mais cette liste ne présente pas de caractère limitatif. Les groupes alkyle sont le plus souvent les substituants préférés.

Les composés oléfiniques seront choisis comme cela est connu en soi en fonction du produit final recherché.

De manière tout à fait préférée, le procédé selon l'invention aura en particulier pour objectif de produire la 2,5-di-tert-octylhydroquinone ou 2,5-bis(1,1,3,3-tétraméthylbutyl)-hydroquinone. De manière pratique, on choisira comme hydroxybenzène l'hydroquinone et comme composé oléfinique le diisobutylène, à savoir α-diisobutylène ou β-diisobutylène ou mélange des deux. La réaction, qui doit conduire au greffage de deux groupements octyles sur l'hydroquinone, est généralement conduite en présence d'au moins 2 moles de diisobutylène pour 1 mole d'hydroquinone. Il va de soi que pour le greffage de deux groupements alkyles par molécule d'hydroxybenzène il faudra utiliser au minimum 2 moles d'oléfines pour 1 mole d'hydroxybenzène, à moins de procéder en 2 fois. Pour le greffage d'un seul groupement alkyle, on se contentera de préférence d'un mole d'oléfine pour 1 mole d'hydroxybenzène.

A titre d'exemples supplémentaires, conformément à l'invention, on pourra aussi produire, à partir du composé aromatique et d'un composé oléfinique approprié, les produits finaux suivants :
- ditertiobutyl-4-nonyl-phénol (oléfine = alcène linéaire en C9 ; composé aromatique = ditertiobutyl-4-phénol)
- ortho-(méthylcyclohexyl)-phénol (oléfine = méthyl-1-cyclohexène-1 ; composé aromatique = phénol)
- diamyle-hydroquinone (oléfine = alcène ramifié en C5 ; composé aromatique = hydroquinone)
- camphényl-phénol (composé oléfinique = camphène ; composé aromatique = phénol).

On a cité un certain nombre de substituants pour l'hydroxybenzène initial et un certain nombre de composés oléfiniques utilisables. Il va toutefois de soi que l'invention n'y est pas limitée et que le procédé selon l'invention pourra être appliqué dans le cas des autres substituants et composés oléfiniques connus de l'art antérieur.

Le composé source de carbocation sera de préférence utilisé à raison de 1 à 10 équivalents, préférentiellement de 2 à 5 équivalents, pour 1 équivalent d'hydroxybenzène.

Pour ce qui est des groupes secondaires ou tertiaires des alcools, éthers et amines, il peut s'agir notamment de groupements ayant de 3 à 30 atomes de carbone, avec éventuellement des cycles et des hétéroatomes, notamment comme cela a été dit à propos des groupes des composés oléfiniques.

Comme alcool tertiaire, on peut citer le tertbutano, comme éther, le méthyltertiobutyléther (NTBE) et comme amine le N-méthyltertbutylamine.

Par acide protonique ou protique fort, on désigne dans la présente invention, un acide ayant un pKa dans l'eau inférieur à -0,1 et, de préférence, inférieur à -1,0.

Le pKa est défini comme la constante de dissociation ionique du couple acide/base, lorsque l'eau est utilisée comme solvant.

Les acides protoniques forts selon l'invention sont préférentiellement choisis parmi l'acide sulfurique et plus préférentiellement les acides sulfoniques, notamment les acides halogénosulfoniques tels que acide fluorosulfonique, acide chlorosulfonique ou acide trifluorométhanesulfonique ; l'acide méthanesulfonique, l'acide éthanesulfonique, l'acide éthanedisulfonique, l'acide benzènesulfonique, les acides benzènedisulfonique, toluènesulfonique, naphtalènesulfonique, naphtalènedisulfonique, l'acide camphorsulfonique, l'acide triflique, l'acide xylène sulfonique.

On préférera l'acide benzènesulfonique en tout premier lieu, ainsi que les acides triflique, méthanesulfonique, camphorsulfonique et paratoluènesulfonique.

On peut encore ajouter au cours du procédé un cosolvant de type hydrocarbure en C₆ à C₁₀, par exemple cyclohexane, heptane, notamment à raison de 1 équivalent volume par rapport à la masse du composé aromatique hydroxylé, ou plus généralement dans un rapport de 0,5 à 3 équivalents.

La réaction peut être conduite sous atmosphère normale. On préfère toutefois travailler en atmosphère de gaz inerte, par exemple sous azote.

Par ailleurs, la réaction pourra être conduite dans une gamme de températures allant de 0 à 100°C, notamment de la température ambiante (environ 25°C) jusqu'à 70°C environ, de préférence entre 50 et 55°C environ, en particulier dans le cas de la synthèse de la 2,5-di-tert-octylhydroquinone.

La réaction est conduite à la pression atmosphérique ou à une pression supérieure, le premier cas étant préféré en particulier lorsque le composé oléfinique est le diisobutylène.

Suivant une modalité avantageuse de l'invention, on charge sous agitation l'hydroxybenzène, le catalyseur, notamment acide protonique fort, et le solvant eau + alcool et l'on amène de préférence le mélange à la température de réaction voulue, puis l'on coule lentement le composé oléfinique, l'alcool ou l'éther dans le mélange sous agitation. De manière plus générale, la coulée peut être effectuée en de 0 à 72 heures.

La durée de réaction peut varier en fonction des conditions réactionnelles, notamment de la température, et des constituants, notamment de l'hydroxybenzène. Elle variera de manière générale entre 1 et 24 heures.

De manière très avantageuse, les produits obtenus et en particulier la 2,5-di-tert-octylhydroquinone peuvent précipiter dans le milieu réactionnel conforme à l'invention et peuvent donc être récupérés, par des méthodes simples de séparation, par exemple par filtration. Il n'est donc pas nécessaire d'ajouter l'étape classique de précipitation dans l'eau.

Cependant, si nécessaire on pourra aussi appliquer les techniques de séparation classiques pour récupérer le produit final.

Un autre avantage important de l'invention est que les jus-mères ou filtrats peuvent être recyclés directement à l'opération suivante.

on pourra aussi effectuer un ou plusieurs lavages à l'eau, de préférence en présence d'un réducteur tel que le dithionique de sodium ou le sulfite de sodium, pour éviter les phénomènes d'oxydation.

L'invention va être maintenant décrite plus en détail à l'aide d'exemples de réalisation non limitatifs.

### Exemple 1 :

Dans cet exemple, on réalise la préparation de la 2,5 ditertoctylhydroquinone.

Dans un réacteur, on introduit 110 g d'hydroquinone (1 mol), 110 ml d'eau, 110 ml de méthanol et 632 g d'acide benzène sulfonique (4 mol). On chauffe à 55°C sous bonne agitation et sous courant d'azote. Par l'intermédiaire d'un système automatisé de coulée, on coule en 1h30 560 g de diisobutylène (5 mol). Puis on laisse la réaction se poursuivre pendant 7 h. On ramène ensuite le milieu réactionnel à température ambiante. La 2,5 ditertoctylhydroquinone précipite. On filtre le milieu réactionnel, puis on lave le précipité avec de l'eau. Le précipité séché est ensuite recristallisé dans le n-hexane. On obtient un rendement de réaction de 74 % en ditertoctylhydroquinone pour un taux de transformation de 88 % de l'hydroquinone. Le produit est analysé et est donné pur à > 99 %.

### Exemple 2 : Synthèse par voie méthanol : comparaison

Même mode opératoire en ajoutant 220 ml de méthanol au lieu de 110 ml de méthanol + 110 ml d'eau. On obtient un rendement de réaction de seulement 39 % en ditertoctylhydroquinone pour un taux de transformation de 80 % de l'hydroquinone.

### Exemple 3 : Synthèse par voie d'eau : comparaison

Même mode opératoire en ajoutant 220 ml d'eau au lieu de 110 ml de méthanol + 110 ml d'eau.

On obtient un rendement de réaction de 25 % en ditertoctylhydroquinone pour un taux de transformation de 38 % de l'hydroquinone.

### Exemple 4 : synthèse de la 2,5-ditertoctylhydroquinone :

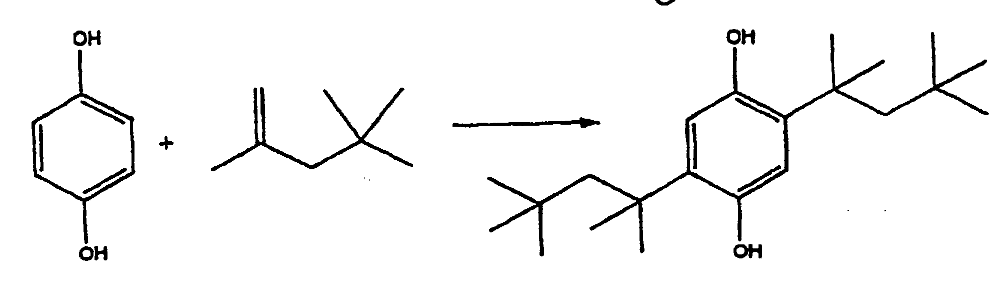

Dans un réacteur, on introduit 110 g d'hydroquinone (1 mol), 110 ml de méthanol, 110 ml d'eau et 632 g d'acide benzène sulfonique (4 mol).

On chauffe à 55°C sous bonne agitation et sous courant d'azote. Par l'intermédiaire d'un système automatisé de coulée, on coule en 2 heures 561 g de diisobutylène (5 mol).

Puis on laisse la réaction se poursuivre pendant 21 heures. Les traitements et purifications sont identiques à précédemment.

On obtient un rendement de réaction de 99% en 2,5 ditertoctylhydroquinone pour un taux de transformation de 99 % de l'hydroquinone.

### Exemple 5 : synthèse de l'hexadécyl hydroquinone

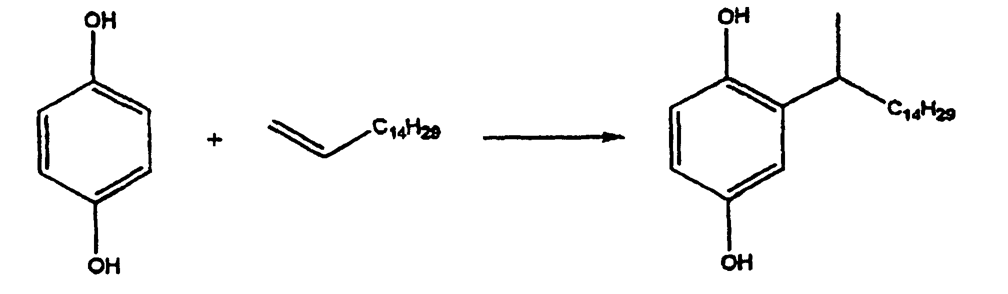

Dans un réacteur, on introduit 110 g d'hydroquinone (1 mol), 110 ml de méthanol, 110 ml d'eau et 632 g d'acide benzène sulfonique (4 mol).

On chauffe à 100°C sous bonne agitation et sous courant d'azote.

Par l'intermédiaire d'un système automatisé de coulée, on coule en 2 heures 1122 g de 1-hexadécène (5 mol).

Puis on laisse la réaction se poursuivre pendant le heures.

Les traitements et purifications sont identiques à précédemment.

On obtient un rendement de réaction de 42 % en mono hexadécyl hydroquinone pour un taux de transformation de 65 % de l'hydroquinone.

### Exemple 6 : synthèse de 3,5-ditertoctyl pyrocatéchol

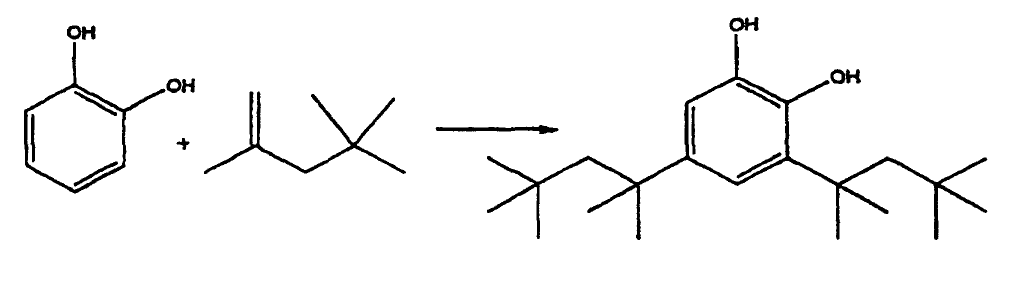

Dans un réacteur, on introduit 110 g de pyrocatéchol (1 mol), 110 ml de méthanol, 110 ml d'eau et 384 g d'acide méthane sulfonique (4 mol).

On chauffe à 55°C sous bonne agitation et sous courant d'azote.

Par l'intermédiaire d'un système automatisé de coulée, on coule en 2 heures 561 g de diisobutylène (5 mol).

Puis on laisse la réaction se poursuivre pendant 5 heures.

Les traitements et purifications sont identiques à précédemment.

On obtient un rendement de réaction de 47% en mono tertoctyl pyrocatéchol et 5% en 3,5-ditertoctyl pyrocatéchol pour un taux de transformation de 55% du pyrocatéchol.

### Exemple 7 : Synthèse de la 2,5-ditertbutylhydroquinone

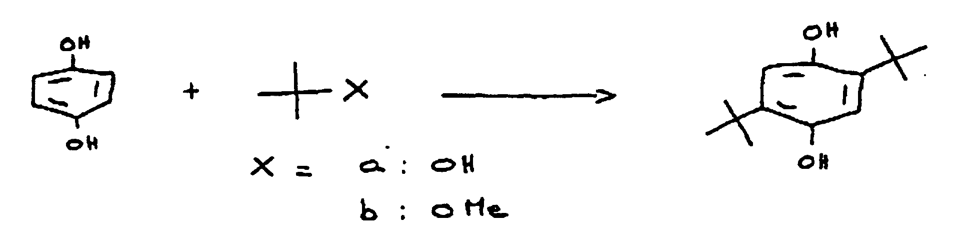

### a : Utilisation du tertiobutanol

Dans un réacteur de 250 ml, on introduit sous courant d'azote 6,2 g d'hydroquinone (0,056 mole) 6,2 ml de méthanol, 6,2 ml d'eau et 35,4 g (0,224 mole, 4 éq.) d'acide benzène sulfonique. Le milieu est porté à 55°C et 20,9 g (0,282 mole, 5 éq.) de tertiobutanol sont additionnés rapidement (5 min). Après 24 heures de maintien, le milieu réactionnel est refroidi à température ambiante et traitement et purification sont réalisés comme précédemment.

on obtient un rendement en produit isolé de 67,5 % pour un taux de transformation de l'hydroquinone de 87 %.

### b : Utilisation du Méthyl TertiobutylEther (MTBE)

Dans un réacteur de 250 ml, on introduit sous courant d'azote 12,4 g (0,113 mole) d'hydroquinone, 49,8 g de MTBE (0,566 mole, 5 éq.) et 21,5 g (0,113 mole, 1 éq.) d'acide benzène sulfonique à 85 %. Le milieu est alors porté à 60°C. Après 5 heures de maintien, 25 ml d'hexane sont additionnés et le milieu réactionnel est refroidi à 20°C, puis filtré pour obtenir 17,8 g de gâteau humide. Celui-ci est placé à l'étuve (35°C) sous vide maximum durant 12 h pour conduire à 14,6 g (rendement de 58,3 % pour un taux de transformation de 80%) de 2,5-ditertbutyl hydroquinone.

### Exemple 8 : synthèse du 3,5-ditertoctyl pyrocatéchol

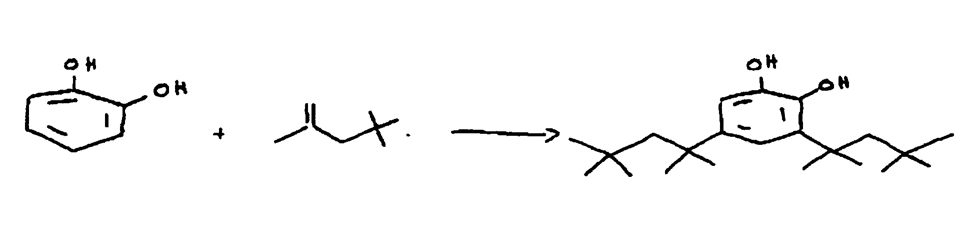

Dans un réacteur de 250 ml, on introduit sous courant d'azote 12,4 g de pyrocatéchol (0,113 mole), 1,3 ml de méthanol, 1,3 ml d'eau, 10 ml de cyclohexane et 2,2 (0,015 mole, 0,13 éq.) d'acide triflique. Après avoir porté le milieu à 55° C, 44 ml de diisobutylène (2,5 éq.) sont additionnés en 2 heures à l'aide d'une ampoule de coulée. Après 19 heures de maintien, 24 ml de diisobutylène (1,36 éq.) sont additionnés en 2 heures et le milieu est à nouveau maintenu 24 heures à 70°C.

On obtient un rendement en produit dosé par HPLC (% de surface) de 74% en 3,5-ditertoctyl pyrocatéchol et 23% de produit de monoalkylation pour un taux de transformation du pyrocatéchol de 99%.

### Exemple 9 : Synthèse du 2,5-di-(1,1,2-triméthyl propyl)hydroquinone.

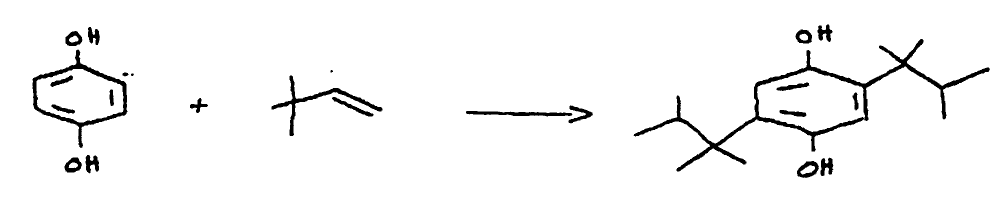

Dans un réacteur de 250 ml muni d'un réfrigérant et d'une ampoule de coulée, on introduit sous courant d'azote 22 g d'hydroquinone (0,2 mole), 5,5 ml de méthanol, 5,5 ml d'eau, et 31,54 g (0,2 mole, 1 éq.) d'acide benzène sulfonique. Après avoir porté le milieu à 50°C, 84,15 g de 3,3-diméthyl 1-butène (1 mole, 5 éq.) sont additionnés en 2 heures à l'aide de l'ampoule de coulée.

Après 25 heures de maintien au reflux de l'alcène (Température d'ébullition = 41°C), on ramène le milieu réactionnel à température ambiante.

Le produit de dialkylation est isolé par cristallisation en additionnant 90 ml d'une solution de méthanol et d'eau dans un rapport 2 pour 1. Le rendement en produit isolé est de 37,2 % pour un taux de transformation de 53%.

## Revendications

1. Procédé de C-alkylation d'un composé aromatique hydroxylé ayant au moins un atome d'hydrogène en ortho ou para par rapport au groupement hydroxyle, dans lequel on met ce composé aromatique en présence d'un acide protonique fort ayant un pKa dans l'eau inférieur à -0,1 et d'un composé conduisant à la formation d'un carbocation en présence de l'acide, la réaction étant conduite en présence d'un solvant formé d'un couple eau/alcool, le rapport eau/alcool (en % volume) du solvant étant compris entre 40/60 et 60/40, de préférence 50/50.

2. Procédé selon la revendication 1, **caractérisé en ce que** le composé formant carbocation en présence de l'acide est choisi parmi le groupe consistant en :
- composés oléfiniques au moins en C3 ;
- alcools secondaires et tertiaires ; et
- éthers ou amines ayant au moins un groupement secondaire ou tertiaire.

3. Procédé selon la revendication 1 ou 2, **caractérisé en ce que** le composé aromatique hydroxylé a pour formule : 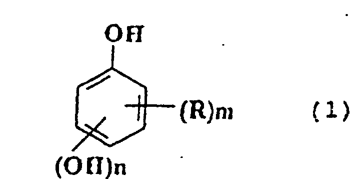 dans laquelle
- n = 0 à 4, notamment 0 ou 1, de préférence 1
- m = 0 à 4, de préférence 0 ou 1
- avec n + m ≤ 4
- R est choisi parmi le groupe consi stant en :
. alkyle linéaire ou ramifié en C1 à C30, notamment en C1 à C6, de préférence en C1 à C4, pouvant éventuellement présenter un ou plusieurs hétéroatomes,
. un ou plusieurs alkyles reliés au noyau aromatique par un hétéroatome, l'alkyle répondant à la définition précédente, par exemple un groupe alcoxy, notamment en C1 à C4, de préférence en C1 à C2,
. un atome d'halogène, de préférence C1, Br.
- 2 groupes R placés sur 2 atomes de carbone voisins pouvant former ensemble et avec les atomes qui les portent 1 cycle aromatique éventuellement substitué.

4. Procédé selon l'un des revendications 1 à 3, **caractérisé en ce que** l'alcool du solvant est un alcool inférieur.

5. Procédé selon la revendication 4, **caractérisé en ce que** l'alcool du solvant est choisi parmi le groupe consistant en méthanol, éthanol, isopropanol, cyclohexanol, éthylèneglycol et mélange d'au moins deux d'entre eux.

6. Procédé selon l'une des revendications 1 à 5, **caractérisé en ce que** le solvant eau + alcool est utilisé à raison de 1 à 10 ml, de préférence de 1 à 5 ml, pour 1 g d'acide.

7. Procédé selon l'une quelconque des revendications 1 à 6, **caractérisé en ce que** le rapport eau + alcool/acide protonique fort est compris entre 1/0,1 et 1/10, de préférence entre 1/0,5 et 1/5, exprimé en volume/masse.

8. Procédé selon l'une quelconque des revendications 1 à 7, **caractérisé en ce que** l'acide protonique fort est un acide sulfonique.

9. Procédé selon l'une quelconque des revendications 1 à 8, **caractérisé en ce que** l'acide est choisi parmi le groupe consistant en acide sulfurique ; acides halogénosulfoniques tels que acide fluorosulfonique, acide chlorosulfonique ou acide trifluorométhanesulfonique ; l'acide méthanesulfonique, l'acide éthanesulfonique, l'acide éthanedisulfonique, l'acide benzèsulfonique, les acides benzènedisulfonique, toluènesulfonique, naphtalènesulfonique, naphtalènedisulfonique, l'acide camphorsulfonique, l'acide triflique, l'acide xylène sulfonique.

10. Procédé selon l'une quelconque des revendications 1 à 9, **caractérisé en ce que** l'acide protonique fort est utilisé à raison de 0,05 à 4 équivalents pour 1 équivalent du composé aromatique de départ.

11. Procédé selon l'une quelconque des revendications 1 à 10, **caractérisé en ce que** le composé oléfinique est un hydrocarbure aliphatique insaturé, linéaire ou ramifié comportant au moins une double liaison de préférence en position α.

12. Procédé selon la revendication 11, **caractérisé en ce que** le nombre d'atomes de carbone de l'hydrocarbure aliphatique est compris entre 3 et 30 atomes de carbone.

13. Procédé selon l'une quelconque des revendications 1 à 10, **caractérisé en ce que** le composé oléfinique est un hydrocarbure monocyclique, carbocyclique, insaturé, ou un hydrocarbure polycyclique comprenant au moins deux carbocyles, pouvant présenter un ou des hétéroatomes dans le cycle et présentant au moins une insaturation endocyclique ou exocyclique.

14. Procédé selon l'une quelconque des revendications 1 à 10 **caractérisé en ce que** le composé oléfinique est un composé terpénique.

15. Procédé selon l'une quelconque des revendications 1 à 14, **caractérisé en ce que** le composé oléfinique est utilisé à raison de 1 à 10 équivalents, de préférence de 2 à 5 équivalents, pour 1 équivalent du composé aromatique de départ.

16. Procédé selon l'une quelconque des revendications 1 à 15, **caractérisé en ce que** le composé aromatique de départ est l'hydroquinone.

17. Procédé selon l'une quelconque des revendications 1 à 16, **caractérisé en ce que** le composé oléfinique est le diisobutylène.

18. Procédé selon l'une quelconque des revendications 1 à 17, **caractérisé en ce que** l'on charge sous agitation le composé aromatique de départ, le catalyseur et le solvant eau + alcool, de préférence l'on amène le mélange à la température de réaction voulue, puis l'on coule le composé oléfinique dans le mélange sous agitation.

## Patentansprüche

1. Verfahren zur C-Alkylierung einer aromatischen Hydroxyverbindung, die mindestens ein Wasserstoffatom in ortho- oder para-Stellug zur Hydroxygruppe trägt, in welchem man die aromatische Verbindung in Gegenwart einer starken Protonensäure, die einen pKa in Wasser unterhalb von 0,1 hat, und einer Verbindung, die in Gegenwart der Säure zu der Bildung eines Carbokations führt, einführt während die Reaktion in Gegenwart eines Lösungsmittels durchgeführt wird, das aus der Kombination Wasser/Alkohol gebildet wird, wobei das Verhältnis Wasser/Alkohol (in Volumenprozent) im Lösungsmittel zwischen 40/60 und 60/40, vorzugsweise 50/50 beträgt.

2. Verfahren nach Anspruch 1, **dadurch gekennzeichnet, dass** die Verbindung, die in Gegenwart der Säure zu der Bildung des Carbokations führt, aus der Gruppe gewählt ist, die besteht aus
- Verbindungen, die zumindest an C3 ungesättigt sind;
- sekundären und tertiären Alkoholen; und
- Ethern oder Aminen, die mindestens eine sekundäre oder tertiäre Gruppierung aufweisen.

3. Verfahren nach einem der Ansprüche 1 oder 2, **dadurch gekennzeichnet, dass** die aromatische Hydroxyverbindung die Formel 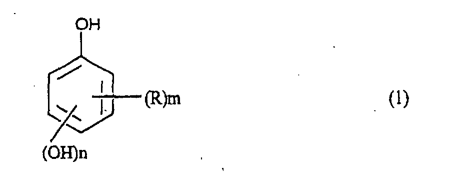 besitzt, in welcher
- n = 0 bis 4, insbesondere 0 oder 1, vorzugsweise 1
- m = 0 bis 4, vorzugsweise 0 oder 1
- mit n + m ≤ 4
- R ausgewählt ist aus der Gruppe, die besteht aus:
. einem linearen oder im Bereich zwischen C1 und C30, insbesondere zwischen C1 und C6, vorzugsweise zwischen C1 und C4 verzweigten Alkan, das gegegebenenfalls ein oder mehrere Heteroatome aufweisen kann.
. einem oder mehreren mit dem aromatischen Kern über ein Heteroatom verbundenenen Alkylresten, die der vorangehenden Definition entsprechen, beispielsweise einer Alkoxygruppe, insbesondere an C1 bis C4, vorzugsweise an C1 bis C2,
. ein Halogenatom, vorzugsweise C1, Br.
- 2 Gruppen R an 2 benachbarten Kohlenstoffatomen, die zusammen und mit den Atomen, die sie tragen, einen möglicherweise substituierten aromatischen Ring bilden.

4. Verfahren nach einem der Ansprüche 1 bis 3, **dadurch gekennzeichnet, dass** der Alkohol des Lösungsmittels ein niederer Alkohol ist.

5. Ein Verfahren nach Anspruch 4, **dadurch gekennzeichnet, dass** der Alkohol des Lösungsmittels ausgewählt ist aus der Gruppe, die aus Methanol, Ethanol, Isopropanol, Cyclohexanol, Ethylenglykol und einem Gemisch aus mindestens zwei dieser Komponenten besteht.

6. Ein Verfahren nach einem der Ansprüche 1 bis 5, **dadurch gekennzeichnet, dass** das Lösungsmittel Wasser plus Alkohol verwendet wird im Verhältnis von 1 bis 10 ml, vorzugsweise von 1 bis 5 ml, auf 1 g Säure.

7. Verfahren nach einem der Ansprüche 1 bis 6, **dadurch gekennzeichnet, dass** das Verhältnis Wasser plus Alkohol/Säure zwischen 1/0,1 und 1/10, vorzugsweise zwischen 1/0,5 und 1/5, bezogen auf Volumen/Masse beträgt.

8. Verfahren nach einem der Ansprüche 1 bis 7, **dadurch gekennzeichnet, dass** die starke Protonensäure eine Sulfonsäure ist.

9. Verfahren nach einem der Ansprüche 1 bis 8, **dadurch gekennzeichnet, dass** die Säure aus der Gruppe ausgewählt ist, die aus Schwefelsäure, Halogensulfonsäuren, wie Fluorsulfonsäure, Chlorsulfonsäure oder Trifluormethansulfonsäure, Methansulfonsäure, Ethansulfonsäure, Ethandisulfonsäure, Benzolsulfonsäure, Benzoldisulfonsäure, Toluolsulfonsäure, Naphthalensulfonsäure, Naphthalendisulfonsäure, Camphersulfonsäure, Trifluormethansulfonsäure und Xylolsulfonsäure besteht.

10. Verfahren nach einem der Ansprüche 1 bis 9, **dadurch gekennzeichnet, dass** 0,05 bis 4 Äquivalente der starken Protonensäure auf 1 Äquivalent der aromatischen Ausgangsverbindung verwendet wird.

11. Verfahren nach einem der Ansprüche 1 bis 10, **dadurch gekennzeichnet, dass** die ungesättigte Verbindung ein ungesättigter, aromatischer, linearer oder verzweigter Kohlenwasserstoff ist, der mindestens eine Doppelbindung, vorzugsweise in alpha-Stellung trägt.

12. Verfahren nach Anspruch 11, **dadurch gekennzeichnet, dass** die Anzahl der Kohlenstoffatome des aliphatischen Kohlenwasserstoffs zwischen 3 und 30 Kohlenstoffatome liegt.

13. Verfahren nach einem der Ansprüche 1 bis 10, **dadurch gekennzeichnet**, das die ungesättigte Verbindung ein monozyklischer, carbozyklischer, ungesättiger Kohlenwasserstoff ist oder ein polyzyklischer Kohlenwasserstoff, der mindestens zwei Carbozyklen enthält, die ein oder zwei Heteroatome im Zyklus enthalten und wenigstens eine endozyklische oder exozkyklische Doppelbindung aufweisen können.

14. Verfahren nach einem der Ansprüche ,1 bis 10, **dadurch gekennzeichnet, dass** die ungesättigte Verbindung ein Terpen ist.

15. Verfahren nach einem der Ansprüche 1 bis 14, **dadurch gekennzeichnet, dass** die ungesättigte Verbindung verwendet wird im Verhältnis von 1 bis 10 Äquivalenten, vorzugsweise von 2 bis 5 Äquivalenten auf 1 Äquivalent an aromatischer Ausgangsverbindung.

16. verfahren nach einem der Ansprüche 1 bis 15, **dadurch gekennzeichnet, dass** die aromatische Ausgangsverbindung Hydrochinon ist.

17. Verfahren nach einem der Ansprüche 1 bis 16, **dadurch gekennzeichnet, dass** die ungesättigte Verbindung Diisobuten ist.

18. Verfahren nach einem der Ansprüche 1 bis 17, **dadurch gekennzeichnet, dass** man die aromatische Ausgangsverbindung, den Katalysator und das Lösungsmittel Wasser plus Alkohol unter Rühren vorlegt, das Gemisch vorzugsweise auf die erwünschte Reaktionstemperatur bringt, dann die olefinische Verbindung unter Rühren zu dem Gemisch gibt.

## Claims

1. Process for C-alkylating a hydroxylated aromatic compound having at least one hydrogen atom in the ortho or para position relative to the hydroxyl group, wherein the aromatic compound is placed in the presence of a strong protonic acid having a pKa in water of less than -0.1 and a compound leading to the formation of a carbocation in the presence of the acid, the reaction being carried out in the presence of a solvent formed by a water/alcohol couple, the water/alcohol proportion (in % by volume) of the solvent being between 40/60 and 60/40, preferably 50/50.

2. Process according to claim 1, **characterised in that** the compound forming the carbocation in the presence of the acid is selected from the group comprising:
- olefinic compounds of at least C3;
- secondary and tertiary alcohols; and
- ethers or amines having at least a secondary or tertiary group.

3. Process according to claim 1 or claim 2, **characterised in that** the hydroxylated aromatic compound has the following formula: 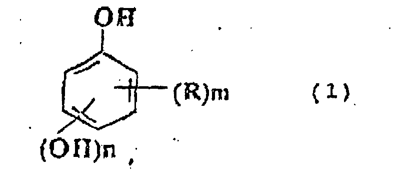 where
- n = from 0 to 4, in particular 0 or 1, preferably 1,
- m = from 0 to 4, preferably 0 or 1,
- with n + m ≤ 4.
- R is selected from the group comprising:
• a linear or branched alkyl of C₁ to C₃₀, in particular C1 to C6, preferably C1 to C4, optionally being able to have one or more heteroatoms,
• one or more alkyls connected to the aromatic core by a heteroatom, the alkyl complying with the above definition, for example, an alkoxy group, in particular C1 to C4, preferably C1 to C2,
• a halogen atom, preferably Cl, Br,
- 2 R groups which are placed on 2 adjacent carbon atoms and which can form, together and with the atoms which carry them, one aromatic ring which is optionally substituted.

4. Process according to any one of claims 1 to 3, **characterised in that** the alcohol of the solvent is a lower alcohol.

5. Process according to claim 4, **characterised in that** the alcohol of the solvent is selected from the group comprising methanol, ethanol, isopropanol, cyclohexanol, ethylene glycol and a mixture of at least two thereof.

6. Process according to any one of claims 1 to 5, **characterised in that** the solvent water + alcohol is used at a proportion of from 1 to 10 ml, preferably from 1 to 5 ml, per 1 g of acid.

7. Process according to any one of claims 1 to 6, **characterised in that** the proportion water + alcohol/strong protonic acid is between 1/0.1 and 1/10, preferably between 1/0.5 and 1/5, expressed as volume/mass.

8. Process according to any one of claims 1 to 7, **characterised in that** the strong protonic acid is a sulphonic acid.

9. Process according to any one of claims 1 to 8, **characterised in that** the acid is selected from the group comprising sulphuric acid; halosulphonic acids, such as fluorosulphonic acid, chlorosulphonic acid or trifluoromethanesulphonic acid; methanesulphonic acid, ethanesulphonic acid, ethanedisulphonic acid, benzenesulphonic acid, benzenedisulphonic, toluenesulphonic, napthalenesulphonic, naphthalenedisulphonic acids, camphorsulphonic acid, triflic acid, xylenesulphonic acid.

10. Process according to any one of claims 1 to 9, **characterised in that** the strong protonic acid is used at a proportion of from 0.05 to 4 equivalents per 1 equivalent of the initial aromatic compound.

11. Process according to any one of claims 1 to 10, **characterised in that** the olefinic compound is a linear or branched, unsaturated aliphatic hydrocarbon comprising at least a double bond, preferably at position α.

12. Process according to claim 11, **characterised in that** the number of carbon atoms of the aliphatic hydrocarbon is between 3 and 30 carbon atoms.

13. Process according to any one of claims 1 to 10, **characterised in that** the olefinic compound is an unsaturated, carbocyclic, monocyclic hydrocarbon, or a polycyclic hydrocarbon comprising at least two carbocycles, capable of having one or more heteroatoms in the ring and having at least one endocyclic or exocyclic unsaturation.

14. Process according to any one of claims 1 to 10, **characterised in that** the olefinic compound is a terpenic compound.

15. Process according to any one of claims 1 to 14, **characterised in that** the olefinic compound is used at a proportion of from 1 to 10 equivalents, preferably from 2 to 5 equivalents, per 1 equivalent of the initial aromatic compound.

16. Process according to any one of claims 1 to 15, **characterised in that** the initial aromatic compound is hydroquinone.

17. Process according to any one of claims 1 to 16, **characterised in that** the olefinic compound is diisobutylene.

18. Process according to any one of claims 1 to 17, **characterised in that** the initial aromatic compound, the catalyst and the water + alcohol solvent are agitated, the mixture is preferably brought to the desired reaction temperature, then the olefinic compound is poured into the mixture and agitated.
